# EUROPÄISCHE PATENTANMELDUNG

(11) **EP 0 904 799 A1**
(43) Veröffentlichungstag der Anmeldung: **31.03.1999**
(21) Anmeldenummer: 97202958.1
(22) Anmeldetag: 26.09.1997
(51) Int. Cl.: A61M 29/02, A61M 25/10

(54) **Ballon für einen Dilatationskatheter mit einem bestimmten Verhältnis von Ballonvolumen zur Querschnittsfläche des Inflationslumens**

(71) Anmelder: Schneider (Europe) GmbH, 8180 Bülach (CH)
(72) Erfinder: Schwager, Michael, 8404 Winterthur (CH); Ghielmetti, Cirillo, 8180 Bülach (CH)

(57) **Zusammenfassung**

Wenn bei einem vorbereiteten Katheter zur intraluminalen Behandlung eines Gefässabschnittes, der einen länglichen Schaft (1) mit einem proximalen und einem distalen Ende (2), einen auf ein maximales Gebrauchsvolumen inflatierbaren Ballon (8) am distalen Ende (2) des Schaftes (1) und ein sich im Schaft (1) erstreckendes und in den Ballon (8) mündendes Inflationslumen (7) aufweist und der mit einem Inflationsmedium (9) zur Beaufschlagung des Ballons (8) mit Druck gefüllt ist, das Inflationsmedium (9) eine Viskosität besitzt geringer als die von Wasser und das Inflationslumen (7) über den grössten Teil der während einer Behandlung im Körper des Patienten liegenden Länge eine Querschnittsfläche aufweist von einem Wert in mm² nicht grösser als das maximale Gebrauchsvolumen des Ballons (8) in mm³ dividiert durch 1 200, wird eine Reduzierung des Gesamtprofils des Katheters erreicht bei gleichzeitiger Erhaltung der erwünschten Deflationszeiten für den Ballon (8).

## Beschreibung

Die Erfindung bezieht sich auf einen vorbereiteten Katheter zur intraluminalen Behandlung eines Gefässabschnittes, der einen länglichen Schaft mit einem proximalen und einem distalen Ende, einen auf ein maximales Gebrauchsvolumen inflatierbaren Ballon am distalen Ende des Schaftes und ein sich im Schaft erstreckendes und in den Ballon mündendes Inflationslumen aufweist und der mit einem Inflationsmedium zur Beaufschlagung des Ballons mit Druck gefüllt ist.

Ballonkatheter werden beispielsweise zum Abdichten in axialer Richtung oder zum radialen Aufweiten von Körpergefässen verwendet. Einen häufigen und typischen Eingriff mit einem Ballonkatheter stellt die perkutane, transluminale Angioplastie im peripheren oder koronaren Blutgefässsystem dar. Dort werden durch Ablagerungen gebildete Verengungsstellen durch Inflatieren eines Dilatationsballons aufgeweitet, um dem Blut wieder ausreichend Strömungsquerschnitt bereitzustellen. Daneben werden Ballonkatheter auch als Transport- und Expansionsmittel für ballonexpandierbare Stents benutzt. Dabei handelt es sich um ein meist metallisches Stützröhrchen, welches während oder nach einer Ballondilatation durch den expandierenden Ballon plastisch verformt wird. Mit Hilfe der plastischen Verformung wird es in den aufgeweiteten Gefässabschnitt implantiert und soll eine Wiederverengung dieses Gefässabschnittes verhindern. Es ist auch bekannt, einen Abschnitt eines Blutgefässes durch zwei auf einem Ballonkatheter hintereinander angeordnete Okklusionsballons abzudichten und in den Zwischenraum ein Medikament zur Behandlung des Gefässes zu infundieren und mögliche Reaktionsprodukte aus dem Zwischenraum abzusaugen.

Ballonkatheter weisen in der Regel ein sich über ihre gesamte Länge oder nur über einen distalen Abschnitt erstreckendes Lumen zur Aufnahme eines im Gefäss vorplatzierten Führungsdrahtes auf, entlang dem der Ballonkatheter bis zum zu behandelnden Gefässabschnitt vorgeschoben wird. Dabei verläuft der Ballonkatheter durch einen ebenfalls vorplatzierten, die Punktionsstelle und den grosslumigen Gefässteil überbrückenden Führungskatheter. Durch das Ringlumen zwischen Ballon- und Führungskatheter wird während der Behandlung Kontrastmittel zur fluoroskopischen Sichtbarmachung der Katheterpositionen und des Behandlungserfolgs gespritzt. Ist der Ballonkatheter in Position gebracht, wird der Ballon über ein Inflationslumen mit physiologischer Kochsalzlösung oder Kontrastmittel gefüllt und unter Druck gesetzt, wodurch der Ballon expandiert.

Die zunehmende Bedeutung minimal-invasiver Eingriffe und die Behandlung immer engerer Blutgefässe erfordern Führungskatheter und damit Ballonkatheter mit immer kleinerem Profil. Dabei müssen Flexibilität sowie Schub- und Torsionsübertragung von Führungsdraht, Ballon- und Führungskatheter ebenso gewährleistet sein, wie geringe Reibung zwischen Führungsdraht und Ballonkatheter. Weiter sind eine hinreichend kurze Ballondeflationszeit und ein genügend grosses Ringlumen für den Kontrastmittelfluss erforderlich.

Bei zu engem Inflationslumen kann das Inflationsmittel nicht mehr schnell genug aus dem Ballon abfliessen. Ein Katheter mit langsamer Ballonentleerung versperrt den Blutstrom länger und verhindert damit z.B. auch die Möglichkeit, während der Behandlung schnell auf eine ischämische Reaktion eines Patienten zu reagieren.

Der Erfindung liegt daher die Aufgabe zugrunde, einen Ballonkatheter der eingangs genannten Art anzugeben, der bei kurzen Deflationszeiten in seinem Gesamtprofil klein ist.

Die Aufgabe wird gemäss der Erfindung gelöst durch einen Katheter der eingangs genannten Art mit den Merkmalen des kennzeichnenden Teils eines der Patentansprüche 1 oder 3. Wenn als Inflationsmedium ein Medium eingesetzt wird mit geringerer Viskosität als die auf Wasser basierenden Inflationsmedien, kann bei im wesentlichen gleicher Deflationszeit das Inflationslumen über den grössten Teil seiner während der Behandlung im Patienten verlaufenden Länge in seinem Querschnitt verringert werden mit grossen Vorteilen für alle anderen Eigenschaften des Katheters. Durch das kleinere Gesamtprofil wird z.B. der Kontrastmittelfluss verbessert, aber ebenso wird auch die Flexibilität und die Knickfestigkeit verbessert, da bei kleineren Schaftdurchmessern die Wandstärke reduziert werden kann. Die durch die Erfindung erreichbare Querschnittsverringerung kann entweder wie in den Ansprüchen angegeben in absoluten Werten angegeben werden oder, wie in anderen Ansprüchen angegeben, abhängig vom maximalen Gebrauchsvolumen des Ballons, der deflatiert werden muss.

Wenn als Inflationsmedium ein Gas eingesetzt wird, kommen die Vorteile der Erfindung besonders gut zur Geltung. Mit Gas gefüllte Ballons haben bis zu etwa dreimal kürzere Deflationszeiten im Vergleich zu Ballons, die mit Flüssigkeit inflatiert wurden. Entsprechend kann in diesem Fall die Querschnittsfläche des Inflationslumens verringert werden.

In einer bevorzugten Ausführungsform der Erfindung ist das Inflationsmedium Kohlendioxid. Bei der Behandlung von Blutgefässen kann im Falle eines undichten oder fehlerhaften Ballons das Blut eine gewisse Menge von Kohlendioxid absorbieren, ohne dem Patienten zu schaden. Da Kohlendioxid ohnehin im Blut transportiert wird, steht auch seine biologische Verträglichkeit im Menschen nicht in Frage.

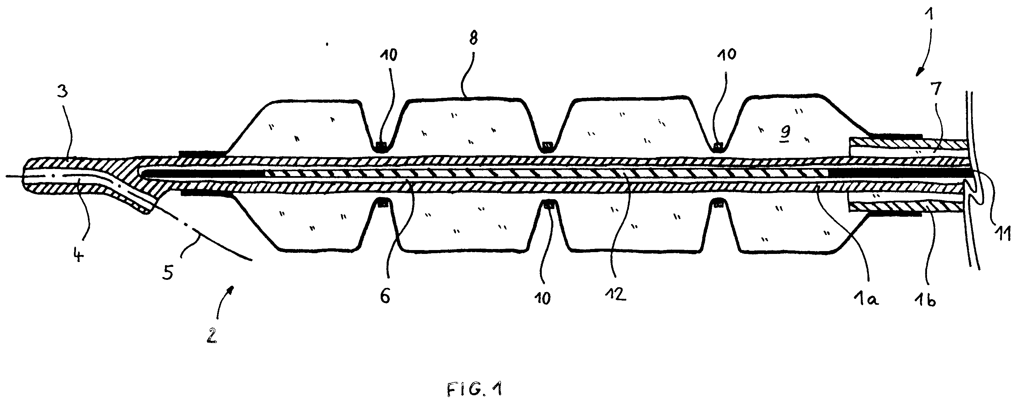

Weitere Eigenschaften eines erfindungsgemässen Katheters ergeben sich aus einem Ausführungsbeispiel, das im folgenden anhand der Zeichnung näher beschrieben wird. Bei dem Beispiel handelt es sich um einen Katheter zur intraluminalen Behandlung eines Gefässabschnittes mit ionisierender Strahlung. Kontrollierte Deflationszeiten bei kleinem Schaftquerschnitt sind hier besonders interessant. Bei längeren Bestrahlungszeiten, wie sie sich aus der nachlassenden Energie des Emitters ergeben können, wird die Belastung des Patienten durch die Unterbrechung des Blutflusses gering gehalten. Die Vorteile der Erfindung zeigen sich jedoch nicht nur bei dieser Anwendung, sondern bei allen Ballonkathetern, deren Deflationszeit und deren Gesamtprofil von Interesse ist, wie z.B. bei den in der Einleitung angegebenen Kathetern, bei Dilatations- oder Okklusionskathetern für die Anwendung in koronaren oder peripheren Blutgefässen oder in der Neurologie und bei anderen Kathetern. In der Zeichnung ist in
- FIG. 1: der distale Abschnitt eines erfindungsgemässen Katheters im Längsschnitt
veranschaulicht.

Gemäss FIG. 1 weist ein erfindungsgemässer Katheter zur intraluminalen Behandlung eines Gefässabschnittes mit ionisierender Strahlung einen dreilumigen Schaft 1 auf, der ein proximales (nicht dargestellt) und ein distales Ende 2 aufweist und als transluminaler Zugang zum Gefässabschnitt dient. Der Schaft 1 ist aus einem Aussenschaft 1a und einem sich in diesem koaxial erstreckenden und distal über diesen hinausragenden Innenschaft 1b aufgebaut. In einer Spitze 3 des Innenschafts 1b ist ein kurzes Führungsdrahtlumen 4 zur Aufnahme eines Führungsdrahtes (nicht dargestellt, Verlauf durch Strichpunktlinie 5 angedeutet) ausgebildet, auf welchen der Katheter zum Voranschieben im Gefässsystem aufgefädelt ist. Ein distal geschlossenes Zentrallumen 6 dient während des Einführens des Katheters zur Aufnahme eines Versteifungsdrahtes (nicht dargestellt), der beim Voranschieben des Schafts 1 axialen Schub auf die Spitze 3 überträgt. Ein zwischen Innenschaft 1a und Aussenschaft 1b verlaufendes, ringförmiges Inflationslumen 7 mündet in einen am distalen Ende 2 des Schafts 1 angeordneten Ballon 8, der über das Inflationslumen 7 mit einem Inflationsmedium 9, beispielsweise Kohlendioxid, gefüllt und dadurch inflatiert wird. Bei annähernd gleicher Entleerungszeit für den Balllon 8 ermöglicht die Verwendung von Kohlendioxid als Inflationsmedium eine Verkleinerung der Querschnittsfläche des Inflationslumens 7 z. B. auf Werte kleiner als 0,300 mm², es sind aber auch schon Querschnittsflächen kleiner als 0,200 mm² hergestellt worden. Das erreichbare Verhältnis zwischen dem maximalen Gebrauchsvolumen des Ballons und der Querschnittsfläche des Inflationlumens bei tolerierbaren Entleerzeiten liegt bei ca 1 200 :1. Um einen dünnen Aussenschaft 1b zu erreichen, wurden teilweise auch die Entleerungszeiten akzeptiert, die sich aus einem Verhältnis 1 600 : 1 ergeben. Der inflatierte Ballon 8 ist von durch Ringelemente 10 gebildete Einschnürungen in mehrere Ballonsegmente unterteilt, wodurch das Zentrallumen 6 auch bei einer Verformung des Innenschaftes 1b radial zentriert ist. Ist der Ballonkatheter positioniert, so wird der Versteifungsdraht aus dem Zentrallumen 6 entfernt und durch einen Quellendraht 11 ersetzt, in den distal eine Quelle 12 ionisierender Strahlung integriert ist. Die Quelle 12 ist beispielsweise ein Filament aus Yttrium 90.

Das als Inflationsmedium verwendete Kohlendioxid steht beispielsweise in Gasflaschen unter einem Druck von z.B. 11 bar zur Verfügung. Der Gasdruck kann über ein Reduzierventil vermindert werden, sodass auch im sterilen Bereich eines Katheterlabors eine Inflationsspritze mit Kohlendioxid gefüllt werden kann. Ballon und Inflationslumen des Katheters werden wie üblich, etwa mit einer Vakuum ziehenden Spritze, entlüftet. Danach kann der Ballon mit dem von der Inflationsspritze aufgenommenen Kohlendioxid inflatiert werden.

### Bezugszeichenliste

- 1: Schaft
- 1a: Innenschaft
- 1b: Aussenschaft
- 2: distales Ende
- 3: Spitze
- 4: Führungsdrahtlumen
- 5: Führungsdrahtverlauf
- 6: Zentrallumen
- 7: Inflationslumen
- 8: Ballon
- 9: Inflationsmedium, Kohlendioxid
- 10: Ringelement
- 11: Quellendraht
- 12: Quelle

## Patentansprüche

1. Vorbereiteter Katheter zur intraluminalen Behandlung eines Gefässabschnittes, der einen länglichen Schaft (1) mit einem proximalen und einem distalen Ende (2), einen auf ein maximales Gebrauchsvolumen inflatierbaren Ballon (8) am distalen Ende (2) des Schaftes (1) und ein sich im Schaft (1) erstreckendes und in den Ballon (8) mündendes Inflationslumen (7) aufweist und der mit einem Inflationsmedium (9) zur Beaufschlagung des Ballons (8) mit Druck gefüllt ist, dadurch gekennzeichnet, dass das Inflationsmedium (9) eine Viskosität besitzt geringer als die von Wasser und dass das Inflationslumen (7) über den grössten Teil der während einer Behandlung im Körper des Patienten liegenden Länge eine Querschnittsfläche aufweist von einem Wert in mm² nicht grösser als das maximale Gebrauchsvolumen des Ballons (8) in mm³ dividiert durch 1 200.

2. Vorbereiteter Katheter nach Anspruch 1,
dadurch gekennzeichnet, dass das Inflationslumen (7) über den grössten Teil der während einer Behandlung im Körper des Patienten liegenden Länge eine Querschnittsfläche aufweist von einem Wert in mm² nicht grösser als das maximale Gebrauchsvolumen des Ballons (8) in mm³ dividiert durch 1 600.

3. Vorbereiteter Katheter nach dem Oberbegriff von Anspruch 1,
dadurch gekennzeichnet, dass das Inflationsmedium (9) eine Viskosität besitzt geringer als die von Wasser und dass das Inflationslumen (7) über den grössten Teil der während einer Behandlung im Körper des Patienten liegenden Länge eine Querschnittsfläche aufweist von höchstens 0,300 mm^{2.}

4. Vorbereiteter Katheter nach Anspruch 3,
dadurch gekennzeichnet, dass das Inflationsmedium (9) eine Viskosität besitzt geringer als die von Wasser und dass das Inflationslumen (7) über den grössten Teil der während einer Behandlung im Körper des Patienten liegenden Länge eine Querschnittsfläche aufweist von höchstens 0,200 mm^{2.}

5. Vorbereiteter Katheter nach einem der vorhergehenden Ansprüche,
dadurch gekennzeichnet, dass das Inflationsmedium (9) ein Gas ist.

6. Vorbereiteter Katheter nach Anspruch 5,
dadurch gekennzeichnet, dass das Inflationsmedium (9) Kohlendioxid ist.
